# EUROPEAN PATENT APPLICATION

(11) **EP 4 512 324 A1**
(43) Date of publication of application: **26.02.2025**
(21) Application number: 24169568.3
(22) Date of filing: 10.04.2024
(51) Int. Cl.: A61B 5/11, A61B 5/16, G16H 10/20, G16H 50/70

(54) **A METHOD FOR PREPARING A TOOL FOR CLASSIFYING PATIENTS WITH NEUROMUSCULAR, PROPRIOCEPTIVE, MOVEMENT AND SENSORIMOTOR DEFICITS INTO DIFFERENT SUBGROUPS BASED ON THEIR MOTOR CONTROL DEFICITS, AND A SYSTEM AND A METHOD FOR CLASSIFYING THESE PATIENTS**

(30) Priority: 12.04.2023 SI 202300051
(71) Applicant: Rosker, Jernej, 3000 Celje (SI)
(72) Inventor: Rosker, Jernej, 3000 Celje (SI); Majcen Rosker, Ziva, 3000 Celje (SI)
(74) Representative: Patentni Biro AF d.o.o.

(57) **Abstract**

The invention belongs to the field of medicine and procedures, technology and equipment/device for analysing data from patient assessments. The method for preparing a tool for classifying patients with neuromuscular, proprioceptive, movement, motor and/or sensorimotor deficits into different clusters and subgroups based on their deficits in motor control comprises:
a) performing at least one test selected in the group comprising spinal sensorimotor control, movement tests, motion kinematics test, joint position sense tests, mobility tests of the spine and hips, balance tests in sitting and standing posture, balance tests in sitting or standing posture with the neck torsion manoeuvre, eye movement tests, different symptoms related to spinal disorders, tests of multiple joint movement variability during predictable and unpredictable movement tasks,
b) principle component analysis,
c) performing at least one clustering method,
d) preparation of a smart learner for classifying individual patients into appropriate clusters with the help of at least one method: Naive Bayes, Random Forest, Support vector machines, Deep Neural network, kNN and Gradient Boosting,
e) assessment of the importance of each input variable in the classification algorithm in order to assess how each variable contributes to the classification of patients to individual clusters.

In addition, the invention incorporates a system and method for classifying patients based on the constructed tool and prediction of treatment outcomes.

## Description

### Field of the invention

The present invention belongs to the field of health and medicine, as well as to the field of procedures, technology, and equipment/device for analyzing and interpreting collected data from patients' functional assessments. In addition, the invention also belongs to the field of structuring data bases for classifying patients into clinically and functionally meaningful and relevant subgroups. The invention relates to a method for preparing a tool for clustering patients with neuromuscular, proprioceptive, movement, motor and/or sensorimotor deficits into different subgroups based on their deficits in motor control, as well as the method for classifying such patients. According to a preferred embodiment the invention is a method for preparing a tool for clustering patients with spine related symptoms and impairments into subgroups, that could provide decision or rational for prevention, treatment, recurrence interventions as well as define the effectiveness of such procedures for alleviating, rehabilitating, and preventing pathology and pathology related signs and symptoms. The invention further relates to a system and a method for classifying patients using the afore-mentioned tool.

### Background of the invention and the technical problem

Spinal disorders are one of the most debilitating chronic non-fatal conditions (Kazeminasab et al., 2022) that have over the years put increased burden on the healthcare system ( Briggs et al., 2018 DOI: 10.2471/BLT. 17.204891; Henschke et al., 2015; DOI: 10.1016/j.mayocp.2014.09.010). They are characterised as significant health decline that often leads to increased risk of developing other chronic health conditions (Briggs et al., 2018). Classifying patients with proprioceptive, movement and/or sensorimotor deficits into sub-groups based on their sensory and movement deficits is needed in order to define appropriate prevention and rehabilitation strategies. The sub-grouping of patients is challenging as the drivers for sensorimotor deficits and impairments are diverse. This is even more demanding if the patients are diagnosed with idiopathic pain or spinal pain due to trauma with or without concussion.

The spine is positioned between the head and the pelvis, consisting of 33 to 34 vertebras and complex muscular-tendinous structures. The spine enables mobility of the human body, the muscular-tendinous system enables movement and stability. Spinal pain has different causes, such as poor posture due to deficits or demands of working environment, degenerating processes in different spinal structures, nerve impairments or impingements leading to radiculopathy or other sensorimotor disfunctions, diseases and traumatic injuries (e.g. whiplash associated disorders, concussion leading to mild traumatic brain injury and others). Modern lifestyle and changes in working habits had led to increase in number of people suffering from idiopathic pain in the cervical, thoracic and lumbar spine, which often leads to chronic states (chronicity). Yearly incidence of spinal pain is between 20%-50%, increasing from year to year. For example, chronic neck pain (pain longer than 3 months) has increased in the last 10 years for 21.1% while low back pain has increased for 17.3%. A 75,7% worldwide increase in neck pain from 1990 to 2017 has been reported (Wu et al., 2021; DOI: 10.21037/atm-20-6868). Cervical and lumbar spine related disorders have been ranked among the top 10, out of 354 diseases with years lived with disability, of which United States is among most effected (Safiri et al., 2020; DOI: 10.1016/j.jmpt.2019.12.008).

The unknown drivers for spine related disorders, which are persistent problem and are responsible for ineffective rehabilitation and prevention, could be identified with this invention. Although current model of care for idiopathic neck pain frequently focuses on alleviating the acute episode of neck pain, one of the biggest problems of our society is recurrence and chronicity ( Sterling et al., 2019; DOI: 10.3390/jcm8081219). There is strong evidence that people with a history of neck pain, even when in remission from pain, present with similar neuromuscular functions (i.e. sensorimotor functions) than during the pain period ( Alalawi et al., 2022; DOI:10.3390/jcm11072042; Devecchi et al., 2021; DOI:10.1371/journal.pone.0249220) suggesting altered neuromuscular functions could be the reason for developing chronicity and recurrence. Chronicity and recurrence present a substantial financial burden for the health care system, which is expected to increase as the rehabilitation of spine related health problems does not meet the desired rehabilitation outcomes. Therefore, more complex assessment with decision making of drivers for treatment of spine related problems is increasingly more important.

Research has provided novel insights into etiology of spine related problems as well as different treatment approaches, however determining the most appropriate rehabilitation strategies for an individual patient remain elusive. Signs and symptoms demand different methods for assessing functional characteristics of patients' functional impairments (oculomotor, neuro-muscular as well as stability, postural balance, movement characteristics of the spine and others) in order to find and direct the focus on most effective prevention, treatment and rehabilitation procedures. Currently, assessments conducted by clinicians and physical therapists in order to recognize above mentioned impairments demand specific and complex theoretical background as well as clinical experience, expertise in evaluation and assessment procedures, interpretation as well as proficiency in applying different assessment approaches. All listed demands might not be reached by all certified specialists since they require additional knowledge and education and present costly and time-consuming effort. Most commonly clinicians' assessments are based on their personal experience as well as using simple tools (e.g. goniometers, inclinometers, laser pointers and others) without incorporating procedures for more in-depth analysis of the data. Consequently, majority of patients with spinal disorders seeking physical therapy fall under the clinical classification of idiopathic pain which is accompanied by heterogenous signs and symptoms. Assessment methods used in clinical practice in general provide moderate to excellent sensitivity and specificity when differentiating patients with spinal disorders from healthy/asymptomatic subjects (Araujo et al., 2020; DOI: 10.1093/ptj/pzaa072; Ernst et al., 2019; DOI: 10.1016/j.msksp.2019.06.006; Majcen Rosker et al., 2022; DOI: 10.1016/j.msksp.2022.102588), however they do not provide the information on the existence or characteristics of subgroups of patents with spinal disorders. Understanding the existence and characteristics of patients' subgroups would be of crucial importance, as it is suggested that current rehabilitation outcomes are suboptimal with high recurrence rates (Bier et al., 2018, DOI: 10.1093/ptj/pzx118; Blanpied et al., 2017 https://doi.org/10.2519/jospt.2017.0302; Chen et al., 2018 DOI: 10.1093/ptj/pzx101; Cohen, 2015; DOI: 10.1016/j.mayocp.2014.09.008).

The technical problem solved by this invention is the design of a tool, system, method and database for clustering and classifying patients with proprioceptive, sensory and movement and/or motor control impairments as well as prediction of rehabilitation outcomes. Especially desirable is the solution for classifying patients with spinal pain disorders into subgroups, which enabled precise and clinically meaningful design of therapies and rehabilitation procedures for alleviating, preventing or rehabilitating spine related disorders.

### Prior art

Patent application US2202128866A1 refers to a method for preparing a treatment plan for patients with chronic spinal pain that involves the following steps:
- gathering direct medical indicators related to chronic pain that are treated as independent variables;
- selection of secondary medical indicators related to chronic pain that are treated as dependent variables;
- selecting medical indicators related to chronic pain that are treated as dependent variables;
- selecting indicators of chronic pain that serve as dependent variable;
- constructing a model of treatment of chronic pain with the application of direct and secondary medical indicators, nonmedical indicators and indicators of chronic pain;
- use/application of chronic pain treatment model so that a mathematical expression of a patient; and
- construction of treatment plan of a patients with chronic pain with the comparison of each mathematical expression of the patient with the selected goal.

Patent application CN104915561A describes a method for analyzing the match between disease and symptoms. Physical signs and data from patient's assessment are used for preparing models of disease, that is used for adjusting the supplementary diagnosis and scheme of rehabilitation. The method is based on preparing a vector for all patients based on the symptoms of disease in testing results that are combined in a matrix. By using the method of principle components, the main component of the matrix is extracted, the data are then adjusted to the main component in order to acquire a function space. For each patient's data are put into the function space and the correlation size is calculated. Based on the correlation calculation the supplementary diagnosis for each patient is prepared.

Patent application CN115281660A describes a system and method for analysis of nonspecific neck pain. Module for measuring and data acquisition is responsible for gathering data on the patient's disease, assessment of angle and preparation of data vector as well as transfer of vector data into a permanent storage module (database). The database is used for storing data such as list of information on the disease, classification model ICF for nonspecific neck, lumbar and thoracic pain, video recording of the test, pictures of muscle anatomy, joints and nerves, different symbol pictures of the software, standard values of the parameter of the human's activity and formula for classification. Module prepares the calculation and comparison with nonspecific classification model of the neck in the database module in line with chosen information on the disease and data of action angle and classification formula and performs actual classification.

### Description of the solution to the technical problem

The invention solves the technical problem of clustering and classifying patients with different neuromuscular, proprioceptive, movement, motor and/or sensorimotor deficits, e.g. patients with spine related disorders, patients with traumatic brain injury and other vestibular and neurological disorders. The technical problem is solved as defined in the independent claim, while preferred embodiments are described in the dependent claims.

The essence of the invention is the method for preparing a tool for clustering patients with spinal disorders, which involves the following steps:
a) performing at least one, preferably two of the tests selected in a group comprising:
   - spinal sensorimotor control,
   - movement tests,
   - motion kinematics test, such as the Butterfly test, the Fly test, figure 8 test, squatting, lunging, hurdle steps, load lifting and similar tests,
   - joint position sense tests,
   - mobility tests of the spine and hips, such as range of motion and kinematic assessments,
   - balance tests in sitting and standing posture,
   - balance tests in sitting or standing posture with the neck torsion manoeuvre, eye movement tests, such as smooth pursuit eye movement tests, fixations, saccades, micro-saccades, pupillometry and others,
   - different symptoms related to spinal disorders, such as questioners, symptom location and provocation, intensity and frequency,
   - tests of multiple joint movement variability during predictable and unpredictable movement tasks,
   preferably the Butterfly test (i.e. the Fly test) and tests of joint position sense, and wherein at least three of the following parameters are analyzed:
   - average time spend on, behind or before the target during each trial, expressed as percentage of each trial duration or similar parameter,
   - smoothness of movement index, i.e. jerk index,
   - amplitude accuracy, calculated as average difference between the target and body parts' actual position expressed in millimetres or degrees,
   - global, absolute, relative or similar repositioning error from each joint movement directions measured in at least 2D space,
   - variable or similar repositioning error from each joint movement directions measured in at least 2D space,
   wherein said parameters may be combined in any combination,
b) Performing a principle component analysis for the tests and set of parameters measured in the previous step, of which further analysis is performed on the principle components that have the eigenvalues greater than 1 and individual parameter weights higher than 0.4 for which size of explained variance is calculated,
c) Performing at least one, preferably three different clustering and at least one prediction method on all measured parameters in step a), that have previously been normalized to the variance of the sample for each parameter individually, in which the preferable but not exclusive methods are (i) Hierarchical Clustering, (ii) k-Means and (iii) Louvain Clustering and (iv) survival analysis with the aim for searching for patient clusters and predicting treatment outcomes,
d) Preferably assessing the quality of the clusters with the Silhouette score
e) Preparation of a smart learner for classifying individual patients into appropriate clusters identified in previous steps, with the help of at least one, preferably multiple methods: Naive Bayes, Random Forest, Support Vector Machines, Deep Neural Network, kNN and Gradient Boosting which are performed in accordance with the algorithms known in the field,
f) Assessment of the importance/contribution of each machine learning model input variable in the classification algorithm is performed preferably with the SHAP values (Shapley additive explanations) where a SHAP value for each value (for each patient and parameter) is presented in order to assess how individual patients classified into each previously defined cluster and how each value contributes to the classification accuracy.

In general, the method of principle components is used for identifying principle components (groups) of parameters measured with previously listed tests, in which usually three components (groups) of parameters are identified for each test and are rarely mixing between tests. The results of clustering methods in spinal pain patients present information on the characteristics of each cluster (how tests and parameters of tests behave in each cluster). It is further analyzed which tests or/and parameters are increased or decreased in each individual cluster of patients. Based on the described analysis, it is identified which parameter components (i.e. above-described deficits) are specific to individual cluster. In addition, these tests and parameters are further used in the predictions of the rehabilitation outcomes for each group individually with methods such as survival analysis or others appropriate methods known in the field.

The principle components of joint position sense test present and comprise three components:
- repositioning error and velocity of the head and neck movements (global, absolute, constant and variable error all measured in all three planes separately and combined as a three-dimensional vector) from the left and right rotation of the head,
- repositioning error of the head and neck (absolute and constant error all measured in all three planes separately and combined as a three-dimensional vector) from flexion and extension of the head,
- repositioning error of the head and neck (variable error measured in all three planes separately and combined as a three-dimensional vector) from the flexion and extension of the head.

The primary components of the Butterfly (the Fly test) comprise three components:
- first component - altered tracking of the easy trajectory, with significant low time spend on target, higher relative time of overshooting and undershooting, increased difference between the target and actual head position (increased amplitude accuracy) and increased smoothness of movement (jerk index),
- second component - altered tracking of the medium and difficult trajectory (parameters in this component are similar as in component 1 only from the medium and difficult trajectory),
- third component - altered tracking of the target at medium and difficult trajectory with increased undershooting of the target and decreased time on the target at medium and difficult level.

Prior to clustering (step C) correlations and relationships comprising above mentioned and other components can be performed. Low correlations between components additionally suggest, that further clustering of patients using machine learning methods is indicated.

Hierarchical clustering may be performed by calculating Euclidean distances or other appropriate methods. Distance between clusters may be further analysed using Ward linkages or other appropriate methods. The k-means method can be performed with multiple re-runs and iterations. For the Louvain clustering the data should be additionally pre-processed such as by principle components. The clustering can be performed using different methods comprising of Euclidean distance metrics with manipulating the number of neighbours and resolution size.

The classification tool is built on the basis of the above-described steps, which comprise algorithms of various machine learning methods. Preferably in the step e) methods are combined using the stacking of all methods applied (using algorithms known in the field), which generates a smart learner that can identify linear and nonlinear characteristics of data and their relations based on new patient assessments methods and classifies them into clusters extracted using above described and other methods for which further rehabilitation approaches are determined.

The choice of the above-described machine learning approaches (Naive Bayes, Random Forest, Support vector machines, Deep Neural network, kNN, Gradient Boosting and others) is determined by their classification performance using indicators comprised of area under the Receiver operating curve (AUC), classification accuracy (CA), true positive rate (sensitivity), true negative rate (specificity) and relation between predicted and actual probability. Preferably, the stacking of above machine learning methods is performed to improve classification accuracy of the final trained system (smart learner).

For each parameter or test (input variables in individual clusters) SHAP values, Gini index, information gain or other similar approaches can be used for evaluating deficits in neck movement for each of the patient clusters separately. The identified tests or parameters are further used for predicting treatment outcomes.

The described method for preparing a tool and the tool itself, prepared by the above-described method according to the invention, can be stored as a local software, cloud-based database or/and other means that can store and analyze data in an appropriate manner comprising of downloadable applications, installed programs, programs or application installed on external units such as external disks, USB keys and others.

The system for clustering and classifying neuromuscular, proprioceptive, movement, motor and/or sensorimotor deficits of patients with spinal disorders according to the presented invention comprises:
a) a display (e.g. virtual reality system, stationary or mobile display) connected to said processor and;
b) a mobile recording system that measures head/eye, limb or body movements, which can be positioned on the head, neck other limbs, trunk or pelvis of the patient, wherein said recording system comprises at least one sensor and/or recording system (e.g. inertial units, virtual reality systems, video oculography/eye trackers, electrooculography, kinematic systems, electromyography, and others);
c) a processor provided with software that is arranged to:
   ∘ Receive (e.g. record) information comprising from the above listed recording and/or measurement units,
   ∘ Track, capture and record the measured components and preferably but not necessarily display them as a cursor, which presents movement of or changes in the measured component,
   ∘ Present the above-described tests on the display (e.g. cursor), that comprises of tasks, where the patient tries to perform free movements, follow the verbal instructions, follow the movement or maintain a certain specific posture, or follow computer generated target trajectory with above-described body parts, muscle contraction or eye movements,
   ∘ Capture and record the displayed patterns, positions, stimuli (e.g. visual, auditory, mechanical, electrical, magnetic etc.) which includes trajectory or position of the cursor movement (e.g. spatial, temporal and other characteristics),
   ∘ Calculate cursor movements and/or changes (e.g. comprising of correlations, deviation analysis and/or cursor movement frequency analysis in different domains, eye movement characteristics, pupillometry, muscle activation, muscle activation patterns and others) between the trajectory of the cursor and computer-generated path/position of the target,
   ∘ Construct the tool based on the above-described types of calculations for classification of patients into subgroups based on the results of above listed mobility, movement control and other tests with the method described above, or pre-prepared tool for patient classification.

The processor connected to a recording or acquisition unit, which comprises a database with information on patient classification. Preferably, the computer program of the processor is arranged to identify different clusters and subgroups of patients, that have a specific functional meaning, with the help of described database as well as preparing prevention, treatment and rehabilitation interventions (e.g. advice, training and exercises, physiotherapy modalities, manual therapy, cognitive-behavioural therapy and others) and prediction of their outcomes.

The system can be designed to comprise two or more recording units, wherein the recording units are placed on different parts of patients' body (e.g. head, neck, trunk, limbs, hips, eyes or others). For example, one recording unit can be placed on the head, neck or limb, while the second recording unit may be placed on the patient's torso.

The method for classifying patients with different neuromuscular, proprioceptive, movement, motor and/or sensorimotor deficits (e.g. patients with spine related disorders, patients with traumatic brain injury and other vestibular and neurological disorders), when the tool or database is prepared, comprises the following steps:
- performing at least one, preferably two of the tests selected in a group comprising:
   - spinal sensorimotor control,
   - movement tests,
   - motion kinematics test, such as the Butterfly test, the Fly test, figure 8 test, squatting, lunging, hurdle steps, load lifting and similar,
   - joint position sense tests,
   - mobility tests of the spine and hips, such as range of motion and kinematic assessments,
   - balance tests in sitting and standing posture,
   - balance tests in sitting or standing posture with the neck torsion manoeuvre, eye movement tests, such as smooth pursuit eye movement tests, fixations, saccades, micro-saccades, pupillometry and others,
   - different symptoms related to spinal disorders, such as questioners, symptom location and provocation, intensity and frequency,
   - tests of multiple joint movement variability during predictable and unpredictable movement tasks,
   preferably the Butterfly test (i.e. the Fly test) and tests of joint position sense, and wherein at least three of the following parameters are analyzed:
   - average time spend on, behind or before the target during each trial, expressed as percentage of each trial duration or similar parameter,
   - smoothness of movement index, i.e. jerk index,
   - amplitude accuracy, calculated as average difference between the target and body parts' actual position expressed in millimetres or degrees,
   - global, absolute, relative or similar repositioning error from each joint movement directions measured in at least 2D space,
   - variable or similar repositioning error from each joint movement directions measured in at least 2D space,
   wherein said parameters may be combined in any combination,
- Use of the above-described tools, which based on the measured data classifies the patient in the appropriate cluster, and
- Determining the most appropriate treatment plan and its projected effectiveness based on the patients' cluster.

The described method for classifying patients can be stored as a computer program, database and/or algorithm that can be stored in any appropriate manner such as downloadable applications, installed programs, programs or application installed on external units such as external disks, UBS keys and other.

The invention will be described in more detail based on exemplary embodiments, results, analysis and figures, which present an example of nonlinear and linear relationship between different parameters of the Butterfly test. The presented embodiment is only one possible example of the invention and is not limiting.

The preferred embodiment of the method for preparing the tool for classifying patients with spine related pain comprises the following steps:
a) Performing the Butterfly test and joint position sense test, analyzing the following parameters:
   ∘ average relative time spend on the target during each trial, expresses as percentage of each trial duration,
   ∘ average relative time spend behind the target,
   ∘ average relative time spend in front of the target,
   ∘ smoothness of movement index,
   ∘ amplitude accuracy, calculated as average difference between the target and limbs actual position expresses in millimeters or degrees,
b) Performing the principal component analysis on the multiple parameters measured in the previous step, wherein further analysis is performed with the principle components presenting with the eigenvalue equal or greater than 1, with the parameters with weights greater than 0.4 and with calculated amount of explained variance,
c) Performing at least one, preferably three clustering methods using all parameters in the step a), that have been normalized on the sample variance for each individual parameter, wherein clustering methods are preferably (i) hierarchical clustering, (ii) k-means, and (iii) Louvain clustering with the goal of finding multiple patient clusters,
d) Verification of clusters, preferably using the silhouette scores,
g) Preparing the smart learner, which classifies patients into appropriate clusters described in the previous step using at least one, preferable multiple machine learning methods: Naive Bayes, Random Forest, Support vector machines, Deep Neural Networks, kNN and Gradient Boosting, which are performed in accordance with the algorithms known in the field, and the machine learning algorithms are stacked in an appropriate manner applied in the field, which generates a smart learner based on the detected linear and nonlinear data characteristics, and
h) Assessment of the importance of each machine learning model input variable in the classification algorithm is performed preferable with the SHAP values (Shapley additive explanations) where a SHAP value for each value (for each patient and parameter/test) is presented in order to assess how individual patient is classified into each previously defined cluster (one or combination of more tests) and how each value contributes to the classification to this specific cluster.

Figure 1 shows the system of the linear and nonlinear sample characteristics and relations, which serve to classify patients into clusters, consequently enabling preparations of adjusted and goal-oriented treatment plans based on the characteristics of each cluster. Such an approach enables more effective treatment of patients with spine related pain.

### Example: Classification of patients with idiopathic neck pain

Ninety-two patients with chronic idiopathic neck pain disorders were enrolled in the study. Patients were recruited from various physical therapy and rehabilitation clinics. Prior to measurements patients were assessed for suitability via a telephone interview by an experienced physical therapist. To be enrolled, all participants had to present with the following inclusion criteria: age between 18 and 65 years, experiencing a minimum of 3 of 10 for pain in the neck on the visual analogue scale. Participants were excluded if they reported: any neurological or vestibular disorders, type II diabetes or diagnosed psychiatric disorders. Furthermore, all participants were required to refrain from taking medication or alcohol 30 hours prior to the study.

Patients were required to mark pain intensity on 10 cm visual analogue scale. Cervicocephalic kinaesthetic sense was assessed using the Butterfly test (Kristjansson et al., 2004; Kristjansson & Oddsdottir, 2010) by an experienced physical therapist. During the Butterfly test, participants were instructed to accurately follow a dynamic unpredictably moving target with their head. Head movements were measured using an inertial measurement unit (NeckSmart, NeckCare Holding ehf., Reykjavik, Iceland). Two repetitions of three different movement paths of increasing difficulty (easy, medium and difficult) were used. Target movement path characteristics and test duration were predefined software.

Accuracy of head and neck movements during the Butterfly test was analysed using the following directional accuracy parameters;
- mean of the relative time spent on the target during each trial expressed as a percentage of total trial time (time-on-target),
- relative time spent behind the target (undershoot),
- time spent in front of the target (overshoot)
- jerk index (smoothness of movement) and
- amplitude accuracy calculated as average mismatch between the target and actual position of the head expressed in millimetres (average amplitude)

Averages for all parameters at all difficulty levels individually were calculated and used for further analysis.

Step 1: In order to identify latent information provided by the fifteen Butterfly test parameters, a principle component analysis (PCA) was applied. As collinearity between parameters exists, Promax rotation with Kaiser Normalization was applied. Only principle components with eigenvalue higher than 1 were used for further analysis. Furthermore, weights of individual parameters were calculated and treated as nonsignificant when they were lower than .4. Those parameters were excluded from individual components. For each individual component the amount of explained variance was calculated. Table 1 presents individual components and their structure. Further the correlation between the individual components was assessed with the correlation coefficient (Table 2).

**Table 1: results of principle component analysis**

| **Parameter** | **Principle components (weights of individual parameters)** | | |
|---|---|---|---|
| | **1** | **2** | **3** |
| AA_e | 0.951 | 0.597 | |
| ToT_e | -0.948 | -0.607 | -0.566 |
| Over_e | 0.857 | 0.683 | |
| Over_m | 0.783 | 0.75 | |
| Sm_e | 0.76 | 0.661 | |
| Und_e | 0.753 | | 0.662 |
| Over_d | 0.532 | 0.852 | |
| Sm_m | 0.53 | 0.849 | |
| AA_m | 0.807 | 0.814 | 0.591 |
| AA_d | 0.721 | 0.8 | 0.549 |
| ToT_d | -0.722 | -0.793 | -0.741 |
| ToT_m | -0.748 | -0.793 | -0.761 |
| Sm_d | 0.515 | 0.772 | |
| Und_m | | | 0.883 |
| Und_d | | | 0.851 |

| | | | |
|---|---|---|---|
| *AA* - *amplitude accuracy, Sm* - *smoothness of movement; Und - undershoot, ToT-time-on-target; Over - overshoot; _e* - *lowest difficulty level of the Butterfly test (easy); _m* - *medium difficulty level of the Butterfly test (medium); _d* - *highest difficulty level of the Butterfly test (difficult).* | | | |

**Table 2: correlation between individual components**

| | **Component** | **1** | **2** | **3** |
|---|---|---|---|---|
| | **1** | 1.00 | .626 | .467 |
| **The Butterfly test** | **2** | .626 | 1.00 | .333 |
| | **3** | .467 | .333 | 1.00 |

| | | | | |
|---|---|---|---|---|
| *Correlation presented with the correlation coefficient (R)* | | | | |

In this step three principle components were identified that differ between each other. The first component presented with highest eigenvalue (8.718) and explained 53.3% of data variance. The second component (2.305 eigenvalue) explained 15.4% of data variance and the third component (1.101 eigenvalue) only 8% of data variance. All three components together explained 76.8% of data variance. The first two components presented with differences in the difficulty levels at which the Butterfly test was performed but consisted of similar parameters. In the first principle component the parameters calculated at the lowest difficulty level were more important as compared to the second principle component where parameters calculated at the highest difficulty level were more important. Both, first and second components consisted of low values of time-on-target and high values od amplitude accuracy, overshoot and smoothness of movement. The third component tended to be dependent on high values of undershoot and low values of ToT at the medium and difficult level of the Butterfly test. In addition, the correlation analysis presented with low to medium correlations between the three components.

Step 2: In the second step, search for clusters of patients suffering from idiopathic neck pain was performed with three different clustering methods; (i) hierarchical clustering, (ii) k-means and (iii) Louvain clustering. In all clustering methods, data were pre-processed using normalization of all sixteen parameters (all Butterfly test parameters and pain level). All fifteen parameters of the Butterfly test and pain level were used for identification of clusters.

Hierarchical clustering was performed by calculating Euclidean distances. Distance between clusters were further analysed using Ward linkages with maximal pruning depth of 10. The cut-off level for determining number of individual clusters was manipulated by changing height ratio in the classification tree. The k-means method was performed with 10 re-runs and 300 maximum iterations. Two to eight clusters were inspected. For the Louvain clustering the data were additionally pre-processed using principle components (limited to 5 principle components). The clustering was performed using Euclidean distance metrics with manipulating the number of neighbours and resolution size.

For further analysis all clustering methods were adjusted to produce two, three or four clusters. The performance of each clustering method producing two, three or four clusters was analysed using the average Silhouette score for all individual clusters and average Silhouette score for all clusters together. When analysing a five-cluster model the average Silhouette score was low and Silhouette plots presented with prominent asymmetries between individual clusters, therefore we decided not to include this model in further analysis.

Step 3: In the next step, the classification accuracy was analysed in order to provide information on how individual patients with neck pain can be classified into previously identified clusters. Performance of six datamining approaches to classify individual patients into specific clusters were compared; Naive Bayes, Random Forest, Support vector machines, Neural network, kNN and Gradient Boosting. In addition, stacking of all applied machine learning approaches was performed in order to study how the classification accuracy could improve if individual datamining approaches are combined. The classification performance was analysed using area under the curve (AUC), classification accuracy (CA), true positive rate (sensitivity) and true negative rate (specificity) (results for each method are presented in table 3).

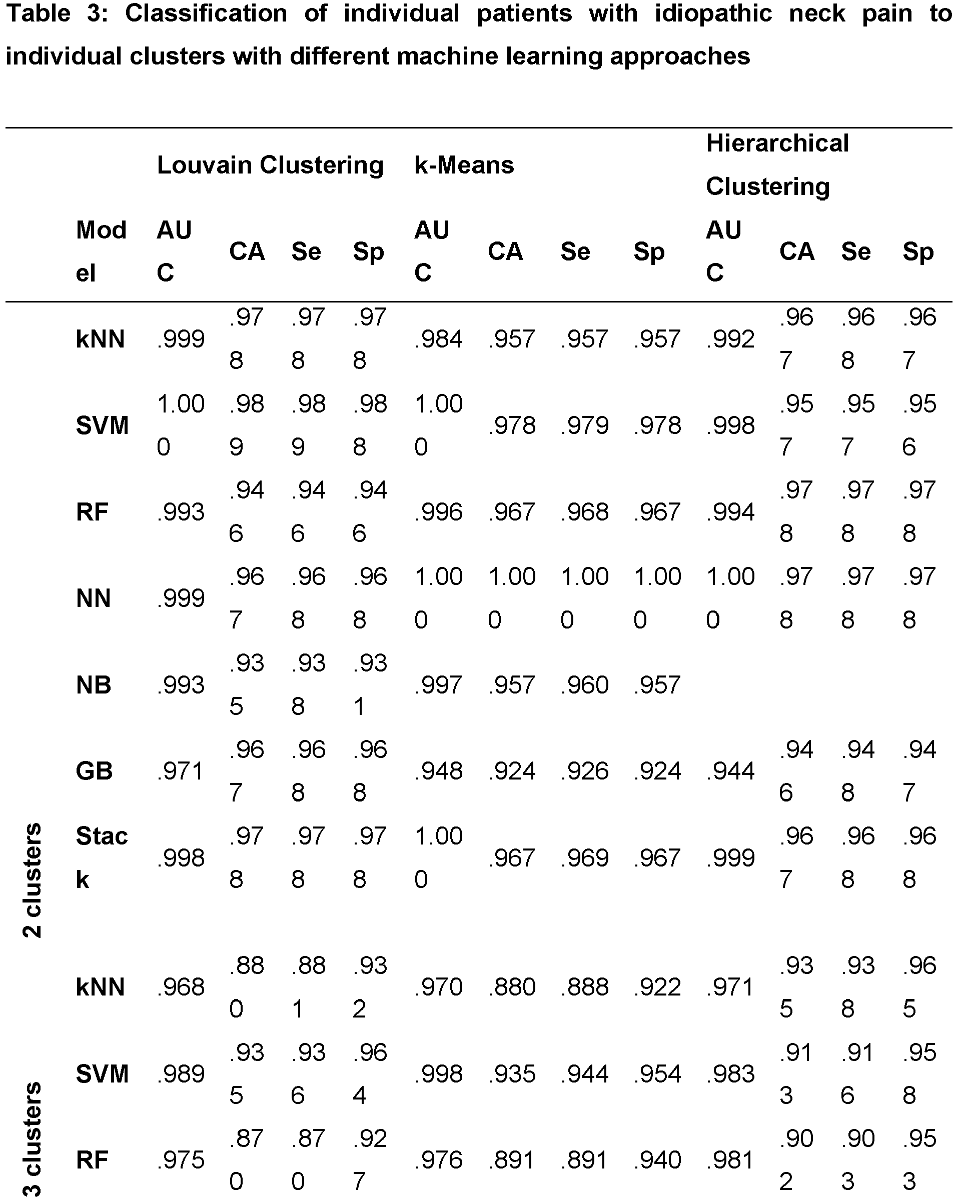

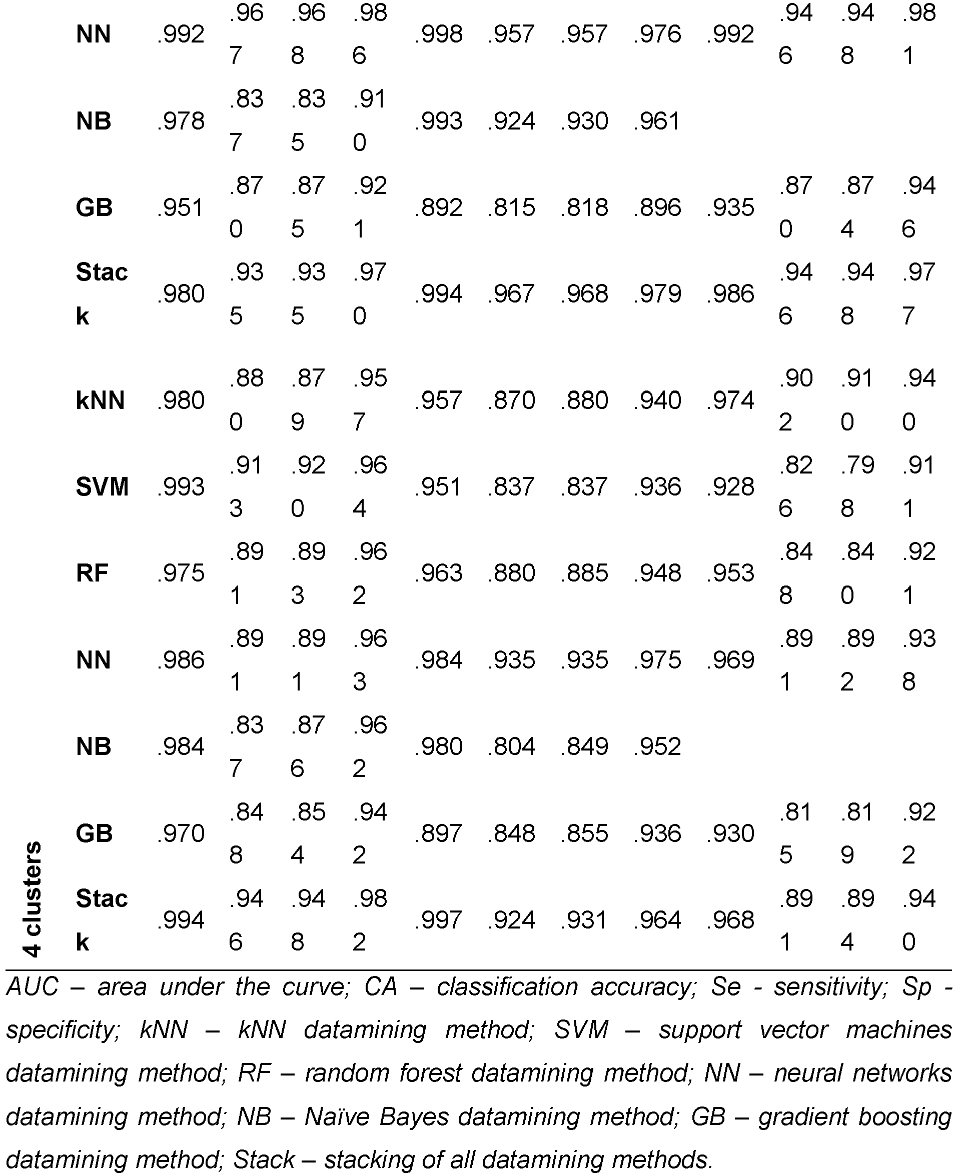

Explanation of the Neural network SHAP library presenting the contribution of individual patients and their scores and derived characteristics of each cluster are described below in Table 4.

**Table 4: cluster characteristics**

| **Cluster** | **Two clusters** | **Three clusters** | **Four clusters** |
|---|---|---|---|
| **C1** | Larger movement deficits (not staying on the target (DA), with larger distance from the target (AA)). | Intermediate movement deficits (not staying on target (DA) but stay close to the target at easy and medium level (AA), primarily undershooting (DA) with low levels of pain). | Smaller movement deficits (stays on target longer (DA), when inaccurate stays closer to the target (AA)). |
| **C2** | Smaller movement deficits (stays on target longer (DA), when inaccurate stays close to the target (AA)). | Larger movement deficits (not staying on target (DA) with large distance from the target (AA), with higher pain levels). | Larger movement deficits at easy level (not staying on target (DA) with large distance from the target (AA), less accurate at the easy difficulty level (DA and AA)). |
| **C3** | | Smaller movement deficits (Stays on target longer (DA), when inaccurate stays closer to the target (AA)). | Larger movement deficits with undershoot (stays on target longer (DA), when inaccurate stays at large distance from the target (AA), primarily undershooting (DA), less accurate at easy difficulty level (DA and AA)). |
| **C4** | | | Larger movement deficits at medium level with undershoot (stays on target |
| longer (DA), when inaccurate stays closer to the target (AA), accurate at easy level (DA), primarily undershoots at medium level (DA), present with higher levels of pain). | | | |

| | | | |
|---|---|---|---|
| *C1* - *cluster 1; C2* - *cluster 2; C3* - *cluster 3; C4 cluster 4; DA* - *directional accuracy parameters group of the Butterfly test; AA* - *amplitude accuracy parameters of the Butterfly test.* | | | |

## Claims

1. A method for preparing a tool for classifying patients with neuromuscular, proprioceptive, movement and sensorimotor deficits into different subgroups based on their motor control deficits, wherein said method comprises at least the following steps:
- performing at least one, preferably two, of the tests selected in a group comprising:
- spinal sensorimotor control,
- movement tests,
- motion kinematics test, such as a Butterfly test, a Fly test, figure 8 test, squatting, lunging, hurdle steps, load lifting and similar,
- joint position sense tests,
- mobility tests of the spine and hips, such as range of motion and kinematic assessments,
- balance tests in sitting and standing posture,
- balance tests in sitting or standing posture with the neck torsion maneuver, eye movement tests, such as smooth pursuit eye movement tests, fixations, saccades, micro-saccades, pupillometry and others,
- different symptoms related to spinal disorders, such as questioners, symptom location and provocation, intensity and frequency,
- tests of multiple joint movement variability during predictable and unpredictable movement tasks,
preferably the Butterfly test (i.e. the Fly test) and tests of joint position sense, and wherein at least three of the following parameters in any combination are analyzed:
- average time spend on, behind or before the target during each trial, expressed as percentage of each trial duration or similar parameter,
- smoothness of movement index, i.e. jerk index,
- amplitude accuracy, calculated as average difference between the target and body parts' actual position expressed in millimetres or degrees,
- global, absolute, relative or similar repositioning error from each joint movement directions measured in at least 2D space,
- variable or similar repositioning error from each joint movement directions measured in at least 2D space,
- performing a principle component analysis for the tests and set of parameters measured in the previous step, of which further analysis is performed on the principle components that have the eigenvalues greater than 1 and individual parameter weights higher than 0.4 for which size of explained variance is calculated,
- performing at least one, preferably three different clustering and at least one prediction method on all measured parameters in step a), that have previously been normalized to the variance of the sample for each parameter individually, wherein the preferred clustering methods are (i) Hierarchical Clustering, (ii) k-Means, (iii) Louvain Clustering, and/or (iv) survival analysis with the aim for searching for patient clusters and predicting treatment outcomes,
- preferably assessing the quality of the clusters with the Silhouette score,
- preparation of a smart learner for classifying individual patients into appropriate clusters identified in previous steps, with the help of at least one, preferably multiple methods: Naive Bayes, Random Forest, Support Vector Machines, Deep Neural Network, kNN and Gradient Boosting,
- assessment of the importance/contribution of each machine learning model input variable in the classification algorithm, wherein the assessment is preferably performed with SHAP values (Shapley additive explanations) where a SHAP value for each value for each patient and parameter is presented in order to assess how individual patients classified into each previously defined cluster and how each value contributes to the classification accuracy.

2. The method for preparing a tool for classifying patients according to claim 1, wherein before step c) a correlation check is performed to check the correlation among said components.

3. The method for preparing a tool for classifying patients according to claim 1 or claim 2, wherein the tool is configured to classify patients with idiopathic neck pain.

4. The method for preparing a tool for classifying patients according to claim 3, wherein in step a) two of the three tests; the Butterfly test, joint position sense or mobility tests are performed.

5. The method for preparing a tool for classifying patients according to claim 4, wherein the primary components of the joint position sense test are characteristic for the grouping in three components:
- repositioning error and velocity of the head and neck movements, which is global, absolute, constant and variable error all measured in all three planes separately and combined as a three-dimensional vector, from the left and right rotation of the head,
- repositioning error of the head and neck, which is absolute and constant error all measured in all three planes separately and combined as a three-dimensional vector, from flexion and extension of the head,
- repositioning error of the head and neck, which is variable error measured in all three planes separately and combined as a three-dimensional vector, from the flexion and extension of the head.

6. The method for preparing a tool for classifying patients according to claim 4 or claim 5, wherein the primary components of the Butterfly test are characteristic for the grouping in three components:
- a first component - altered tracking of the easy trajectory, with significant low time spend on target, higher relative time of overshooting and undershooting, increased difference between the target and actual head position (increased amplitude accuracy) and increased smoothness of movement (jerk index),
- a second component - altered tracking of the medium and difficult trajectory, wherein the parameters in this component are similar as in component 1 only from the medium and difficult trajectory,
- a third component - altered tracking of the target at medium and difficult trajectory with increased undershooting of the target and decreased time on the target at medium and difficult level.

7. The method for preparing a tool for classifying patients according to any of the preceding claims, wherein in step c) more than one clustering method is used.

8. The method for preparing a tool for classifying patients according to any of the preceding claims, wherein in step c) three different clustering methods are used, namely Hierarchical Clustering, k-Means and Louvain Clustering.

9. The method for preparing a tool for classifying patients according to the claim 8, wherein the hierarchical clustering is performed by calculating Euclidean distances between individual values of parameters of analysed patients, followed by analysis of calculated distances using Ward linkages with maximal pruning depth of 10, wherein the k-means method is performed with 10 re-runs and 300 maximum iterations, while Louvain clustering comprises data pre-processing using principle components.

10. The method for preparing a tool for classifying patients according to any of the preceding claims, wherein in step e) trained systems are combined using a stacking method, which generates a smart learner arranged to identify linear and nonlinear characteristics of data and their relations based on new patient assessments methods and classifies them into previously recognized clusters.

11. A computer program, a computer database and/or executable instructions saveable in any suitable manner, such as downloadable applications, downloadable programs, programs on external units, **characterized in that** it comprises the method according to any of the preceding claims.

12. A system for classification of patients with neuromuscular, proprioceptive, movement and sensorimotor deficits, wherein said system comprises:
- a display connected to a processor;
- a wearable mobile recording system arranged to measure head/eye, limb or body movements, which can be positioned on the head, neck other limbs, trunk or pelvis of the patient, wherein said recording system comprises at least one sensor and/or recording system;
- a processor comprising software is arranged to:
∘ receive information comprising from the above listed recording and/or measurement units,
∘ track, capture and record the measured components and preferably but not necessarily display them as a cursor, which presents movement of or changes in the measured component,
∘ present the above-described tests on the display (e.g. cursor), that comprises of tasks, where the patient tries to perform free movements, follow the verbal instructions, follow the movement or maintain a certain specific posture, or follow computer generated target trajectory with above-described body parts, muscle contraction or eye movements,
∘ capture and record the displayed patterns, positions, stimuli, which includes trajectory or position of the cursor movement,
∘ calculate cursor movements and/or changes between the trajectory of the cursor and computer-generated path/position of the target,
∘ construct the tool based on the above-described types of calculations for classification of patients into subgroups based on the results of above listed mobility, movement control and other tests with the method described above, or pre-prepared tool for patient classification.

13. The system according to the preceding claim, wherein the device for tracking movement comprises two sensors, wherein one sensor is placeable on the head, neck or limb of a patient, and the second sensor is placeable on the patient's torso.

14. A method for classification of patients with different neuromuscular, proprioceptive, movement, motor and/or sensorimotor deficits, said method comprising the following steps:
- performing at least one, preferably two of the tests selected in a group comprising:
- spinal sensorimotor control,
- movement tests,
- motion kinematics test, such as a Butterfly test, a Fly test, figure 8 test, squatting, lunging, hurdle steps, load lifting and similar,
- joint position sense tests,
- mobility tests of the spine and hips, such as range of motion and kinematic assessments,
- balance tests in sitting and standing posture,
- balance tests in sitting or standing posture with the neck torsion maneuver, eye movement tests, such as smooth pursuit eye movement tests, fixations, saccades, micro-saccades, pupillometry and others,
- different symptoms related to spinal disorders, such as questioners, symptom location and provocation, intensity and frequency,
- tests of multiple joint movement variability during predictable and unpredictable movement tasks,
- preferably the Butterfly test (i.e. the Fly test) and tests of joint position sense, and wherein at least three of the following parameters are analyzed in any combination:
- average time spend on, behind or before the target during each trial, expressed as percentage of each trial duration or similar parameter,
- smoothness of movement index, i.e. jerk index,
- amplitude accuracy, calculated as average difference between the target and body parts' actual position expressed in millimetres or degrees,
- global, absolute, relative or similar repositioning error from each joint movement directions measured in at least 2D space,
- variable or similar repositioning error from each joint movement directions measured in at least 2D space,
- using the tool prepared with the method according to any claim from 1 to 10 or the program according to claim 11, which based on obtained results classifies the patient into a suitable cluster, and
- defining the most suitable rehabilitation based on the determined cluster to which the patient belongs.

15. A computer program, a computer database and/or executable instructions saveable in any suitable manner, such as downloadable applications, downloadable programs, programs on external units, **characterized in that** it comprises the method according to the preceding claim.
